# EUROPÄISCHE PATENTSCHRIFT

(11) **EP 3 171 846 B1**
(45) Veröffentlichungstag und Bekanntmachung des Hinweises auf die Patenterteilung: **04.08.2021**
(21) Anmeldenummer: 15734314.6
(22) Anmeldetag: 18.06.2015
(51) Int. Cl.: A61J 1/14

(54) **VERSCHLUSS FÜR PHARMAZEUTISCHE GEBINDE SOWIE VERFAHREN ZUM VERSCHLIESSEN EINES FLÄSCHCHENS**
CLOSURE FOR PHARMACEUTICAL CONTAINERS AND METHOD FOR SEALING A BOTTLE
FERMETURE POUR CONTENANTS PHARMACEUTIQUES ET PROCÉDÉ DE FERMETURE D'UN FLACON

(30) Priorität: 22.07.2014 DE 102014110327
(43) Veröffentlichungstag der Anmeldung: 31.05.2017
(73) Patentinhaber: OSARTIS GmbH, 64839 Münster (DE)
(72) Erfinder: DEUSSER, Stefan, 63791 Karlstein (DE); ANDERS, Rike, 61440 Oberursel (DE); SATTIG, Christoph, verstorben (DE)
(74) Vertreter: Blumbach · Zinngrebe Patentanwälte PartG mbB
(86) Internationale Anmeldenummer: PCT/EP2015/063727
(87) Internationale Veröffentlichungsnummer: WO 2016/012165

(56) Entgegenhaltungen:
- EP-A1- 0 569 835
- EP-A1- 0 569 835
- EP-A2- 1 435 254
- EP-A2- 1 435 254
- WO-A1-2014/104027
- BE-A- 538 395

## Beschreibung

### Gebiet der Erfindung

Die Erfindung betrifft einen Verschluss für Flaschen bzw. Fläschchen sowie ein Verfahren zum Verschließen eines Fläschchens. Insbesondere betrifft die Erfindung einen Verschluss, welcher für hermetisch verschlossene Gebinde im medizintechnischen- und pharmakologischen Bereich vorgesehen ist, im speziellen für die Flüssigkomponente eines Knochenzements.

### Hintergrund der Erfindung

Im Pharmabereich werden flüssige Stoffe häufig in Glasampullen aufbewahrt. Derartige flüssige Stoffe, wie insbesondere die Flüssigkomponente eines Knochenzements, können leicht flüchtig chemisch aggressiv oder giftig sein. Besonders geeignet für derartige Flüssigkeiten sind daher Glasampullen, welche chemisch beständig und gleichzeitig derart gasdicht und flüssigkeitsdicht sind, dass allenfalls in sehr geringer Menge Komponenten der in der Flasche gelagerten Flüssigkeit aus dem Gebinde austreten oder dass Gase, wie beispielsweise Sauerstoff, in oder aus dem sich bildenden Gebinde gelangen, wenn dieses aus einem zugeschmolzenen Glaskörper wie zum Beispiel einer Ampulle besteht.

So ist es möglich, beispielsweise auch die Monomerkomponente eines Knochenzements, wie beispielsweise Methylmethacrylat, über mehrere Jahre in einer derartigen Ampulle verlustfrei aufzubewahren. Dieses erfolgt in Glasampullen, deren Kopf vor der Entleerung abgebrochen werden muss.

Diese Brechampullen sind aber in der Handhabung nicht optimal. So kann es zum Ablösen von Splittern kommen, wobei Verletzungen des Anwenders oder Verunreinigungen der Flüssigkeit möglich sind. Weiter ist die Entnahme der Flüssigkeit, beispielsweise mittels einer Spritze, umständlich und birgt ein erhöhtes Risiko, dass die Ampulle derart schräg gehalten wird, dass Flüssigkeit heruntertropft oder dass die Entnahme der Flüssigkeit von der Menge her ungenau ist oder es zu Verletzungen kommen kann.

Weiter bekannt sind Gebinde, welche mit einem Septum abgedichtet sind. Ein derartiges Septum besteht in der Regel aus einer Elastomerschicht, welche mit einem Crimpverschluss aus Aluminium auf den Kragen eines Fläschchens gebördelt ist.

Derartige Gebinde haben den Vorteil, dass sie beispielsweise mit einer Hohlnadel zur Flüssigkeitsentnahme eingestochen werden können. Das Septum verschließt sich nach dem Herausziehen der Hohlnadel derart, dass allenfalls eine geringe Gefahr besteht, dass unbeabsichtigt weiter Flüssigkeit aus dem Gebinde austritt oder fremdkörper in das Gebinde eintreten können.

Es hat sich aber gezeigt, dass derartige Verschlüsse auch bei Verwendung von hochwertigen mehrlagigen Dichtungen für viele Anwendungen nicht die nötige Dichtigkeit aufweisen.

Es kommt zu Evaporationen des flüssigen Inhaltes über die Lagerzeit.

So kommt es beispielsweise bei der Monomerkomponente eines Knochenzements, wie beispielsweise Methylmethacrylat, zum Ausgasen und damit zu einem Flüssigkeitsverlust. Neben einer ungenauen Dosierung kann dies, sofern Flüssigkomponente und Festkomponente nebeneinander in einer Verpackung aufbewahrt sind, auch zur unbeabsichtigten teilweisen Reaktion der Pulverkomponente führen.

Ein weiteres Problem ist eine nicht hinreichende Gasdichtigkeit in Bezug auf Gase, die in das Fläschchen eindringen und mit dem Inhalt reagieren könnten. Dies kann auch ein Problem sein, wenn das Gebindeäußere mittels Gas sterilisiert wird. Zum Sterilisieren der Verpackung wird insbesondere hochgiftiges Ethylenoxid verwendet, welches auch in geringer Menge keinesfalls in das Gebinde gelangen darf.

EP 1 435 254 offenbart einen Verschluss umfassend eine auf ein Fläschchen aufsetzbare Kappe aus einem dielektrischen Material und eine Metallfolie, über welche eine elastische Dichtung eingesetzt ist.

### Aufgabe der Erfindung

Der Erfindung liegt demgegenüber die Aufgabe zugrunde, die genannten Nachteile des Standes der Technik zumindest zu reduzieren.

Es ist insbesondere eine Aufgabe der Erfindung, ein sicher handhabbares Gebinde zur Aufbewahrung von Flüssigkeiten bereitzustellen, bei welchem der Verschluss eine hohe Gasdichtigkeit aufweist.

### Zusammenfassung der Erfindung

Die Aufgabe der Erfindung wird bereits durch einen Verschluss nach Anspruch 1 sowie durch ein Verfahren zum Verschließen eines Fläschchens nach Anspruch 12 gelöst.

Bevorzugte Ausführungsformen und Weiterbildungen der Erfindung sind dem Gegenstand der Unteransprüche zu entnehmen.

Die Erfindung betrifft einen Verschluss, welcher insbesondere zur Verwendung für medizinischpharmazeutische Gebinde vorgesehen ist.

Der Verschluss umfasst eine auf ein Fläschchen aufsetzbare Kappe aus einem dielektrischen Material, insbesondere aus einem Polymer.

Unter einem Fläschchen im Sinne der Erfindung werden insbesondere Glasfläschchen verstanden, welche einen Kragen aufweisen.

Es ist aber auch denkbar, die Erfindung für andere Arten von Fläschchen zu verwenden, so auch für größere Gebinde. Denkbar ist unter Umständen auch die Erfindung für Fläschchen aus anderen Materialien als aus Glas zu verwenden, insbesondere für Kunststofffläschchen. Das Fläschchen sollte aber aus einem dielektrischen Material, also nicht aus einem elektrisch leitenden Material wie Metall, bestehen. Der Verschluss umfasst eine in die Kappe eingesetzte Metallfolie, welche im angebrachten Zustand des Verschlusses auf einem Rand des Fläschchens sitzt. Als Metallfolie wird vorzugsweise eine Aluminiumfolie verwendet. Diese sitzt in bevorzugter Weise auf dem Kragen eines Fläschchens.

Zumindest der Bereich der Metallfolie, welcher auf dem Rand des Fläschchens sitzt, ist mit einem Kleber, insbesondere einem adhäsivem Polymer versehen, welcher über die Metallfolie induktiv durch Erwärmung des Folienmaterials aufgeschmolzen werden kann.

Derartige über einen induktiv erwärmbaren Schmelzkleber aufbringbare Aluminiumsiegel sind grundsätzlich aus dem Stand der Technik bekannt.

Es handelt sich insbesondere um Aluminiumfolien, welche mit einem nach FDA 21 CFR § 177.1330 zulässigen thermoplastischen Polymer beschichtet sind.

Über der Metallfolie, also zwischen der Oberseite des Verschlusses, und der Metallfolie ist eine elastische Dichtung eingesetzt, über welche die Metallfolie nach dem Aufsetzen der Kappe auf das Fläschchen bereits dichtend auf den Rand des Fläschchens gedrückt ist, und zwar ohne dass zu diesem Zeitpunkt bereits das Fläschchen induktiv durch Erwärmung der Metallfolie und Aufschmelzen des Klebers versiegelt wurde.

Die Erfindung betrifft also einen Verschluss mit einem induktiv aufbringbaren Aluminiumsiegel, welcher bereits mit Aufbringen des Verschlusses derart dichtet, dass dieser im Normalfall flüssigkeitsdicht ist.

Weiter ist die Oberseite der Kappe mit zumindest einer Aussparung versehen, durch welche die elastische Dichtung und die Metallfolie durchstoßen werden kann.

Auch ist die Verwendung sogenannter "Flip off" Kappen welche eine abnehmbare zusätzliche Abdeckung über der Aussparung aufweisen denkbar.

Die elastische Dichtung sorgt für eine dichte Anlage der Metallfolie beim induktiven Siegeln. Die Anlagekraft kann auch durch das Siegelwerkzeug verstärkt oder begrenzt werden.

Weiter wirkt die elastische Dichtung als Septum.

Durch die Erfindung wird mithin ein Gebinde bereitgestellt, dessen Verschluss mit einer Hohlnadel durchstoßen werden kann, um Flüssigkeit zu entnehmen, welches im Normalfall nach Entfernen der hohlen Nadel wieder zumindest flüssigkeitsdicht ist.

Die Erfinder haben herausgefunden, dass erst durch ein Aluminiumsiegel, welches mit einem induktiv erwärmbaren Aufschmelzkleber versehen ist, eine hinreichende Dichtigkeit erzielt werden kann derart, dass leicht flüchtige Stoffe, wie beispielsweise Methylmethacrylat, ein organisches Lösemittel, lange aufbewahrt werden können, ohne zu entweichen sowie derart, dass nicht die Gefahr besteht, dass beim Sterilisieren mit einem Gas wie Ethylenoxid dieses in das Gebinde gelangt.

Der Verschluss ist als Prellverschluss ausgebildet. Dies sind Verschlüsse, die im Regelfall auf einen Kragen gedrückt werden, wobei der Verschluss aus einem Polymer besteht, welches derart elastisch ist, dass es so weit gedehnt werden kann, um über die größte Mündungsstelle des Fläschchens geschoben zu werden. Vorzugsweise haben derartige Kunststoffe eine Streckgrenze von mindestens 5 % nach ISO 527-2.

Die Kappe ist vorzugsweise derart ausgebildet, dass diese nicht zerstörungsfrei, oder nur mit speziellen Werkzeug, entfernt werden kann.

So können beispielsweise Krallen, die unterhalb des Kragens eines Fläschchens einrasten, auf einer Seite abgeschrägt sein, um über den Kragen zu gleiten, wohingegen die gegenüberliegende Seite derart am Kragen anliegt, dass die Kappe zerstört wird, wenn diese mit Gewalt abgezogen wird. Bei einer bevorzugten Ausführungsform der Erfindung ist die elastische Dichtung gegenüber der Metallfolie verdrehbar. Dichtung und Metallfolie liegen also im nicht montierten Zustand lose übereinander. Denkbar ist aber auch, Metallfolie und elastische Dichtung beispielsweise mit einem Wachs zu versehen, welches beim induktiven Verschmelzen abschmilzt und sodann eine Verdrehung von Metallfolie und elastischer Dichtung zueinander zulässt. Dadurch, dass Metallfolie und elastische Dichtung im montierten Zustand nicht fest miteinander verbunden, wie beispielsweise verklebt, sind, besteht nicht die Gefahr, dass beim Drehen der Kappe die Metallfolie beschädigt wird oder sich vom Gebinderand ablöst.

Denkbar ist auch, die elastische Dichtung auf der an die Metallfolie angrenzenden Seite mit einer zusätzlichen elastischen Schicht, wie beispielsweise einer Schicht aus Polytetraflurethylen oder ähnlichem zu versehen.

Um als Septum zu wirken sowie um hinreichend Andruckkraft gegenüber der Metallfolie zu entwickeln, beträgt bei der Erfindung die Dicke der elastischen Dichtung 0,1 bis 5 mm, besonders bevorzugt 2 bis 4 mm.

Für die elastische Dichtung gibt es eine Vielzahl denkbarer möglicher Materialien, insbesondere wird Silikon verwendet. Es wird insbesondere ein Material verwendet, welches eine Härte nach Shore A von 20 bis 80°, insbesondere von 30 bis 60° aufweist.

Bei einer Weiterbildung der Erfindung ist die Kappe und/oder die elastische Dichtung zumindest teilweise aus einem transparenten Material ausgebildet.

Diese Ausführungsform der Erfindung ermöglicht insbesondere eine optische Qualitätskontrolle derart, dass nunmehr das Aluminiumsiegel sichtbar ist und optisch, insbesondere auch mit einer Kamera, kontrolliert werden kann, ob dieses über den gesamten Umfang auf dem Rand anliegt oder ob dieses Falten oder Knicke hat.

Bei einer Ausführungsform der Erfindung umfasst die Kappe Krallen zum Verrasten auf einem Kragen des Fläschchens, wobei die Krallen insgesamt über einen Umfang der Kappe von maximal 180° verteilt sind.

Diese Ausführungsform der Erfindung ermöglicht eine vereinfachte Herstellung, insbesondere eines Prellverschlusses, bei welchem die Kappe nach dem Spritzen gedreht und aus der Form ausgeworfen werden kann..

Beispielsweise können drei gleichmäßig über den Umfang verteilte Krallen vorgesehen sein, wobei jede Kralle einen Umfangswinkel von maximal 60° einnimmt.

Bei einer weiteren Ausführungsform der Erfindung umfasst die Kappe Krallen, wobei auf der Oberseite der Kappe über den Krallen Ausnehmungen vorgesehen sind. So können die Krallen der Spritzgussform mittels eines Schiebers in einfacher Weise geformt werden.

Denkbar ist aber auch die Ausnehmungen zum Einsetzen eines Entformungswerkzeugs zu verwenden oder eines Werkzeugs, mit welchem der Verschluss montiert wird.

Bei einer bevorzugten Ausführungsform der Erfindung dienen die Krallen gleichzeitig einem Halten von Metallfolie und elastischer Dichtung.

Aufgrund ihrer Elastizität können die elastische Dichtung sowie die Metallfolie in die Kappe gedrückt werden, sind sodann aber durch die Krallen daran gehindert, herauszufallen. Dies ermöglicht eine Andienung des gesamten Verschlusses über nur eine Station.

Die Erfindung betrifft insbesondere Gebinde, welche mit einem Monomer für eine Autopolymerisation gefüllt sind, insbesondere mit einem Knochenzementmonomer.

Beispielsweise betrifft die Erfindung auch die Verwendung des Gebindes für eine Mischvorrichtung für Knochenzement.

Eine derartige Mischvorrichtung für Knochenzement ist beispielsweise aus der Offenlegungsschrift WO 2010/105807 A1 bekannt. In einer derartigen Vorrichtung wird das Monomerfläschchen, beispielsweise durch Einstechen mit einer Hohlnadel, entleert, wobei die Flüssigkeit in einen Mischraum eintritt und dort mit der Festkomponente des Knochenzements vermischt wird.

Die Erfindung betrifft des Weiteren ein Verfahren zum Verschließen eines Fläschchens mit einem vorstehend beschriebenen Verschluss, wobei das Fläschchen zunächst befüllt wird, sodann der Verschluss aufgesetzt wird und im Anschluss mittels Induktion die Metallfolie erwärmt wird, so dass der Kleber aufschmilzt und die Metallfolie mit einem Rand des Fläschchens stoffschlüssig verbindet. Insbesondere Prellverschlüsse können automatisiert in kurzer Zeit aufgebracht werden, was den Anlagendurchsatz steigert.

Das Versiegeln der Metallfolie durch induktives Erwärmen kann sowohl direkt nach dem Aufsetzen als auch zu einem späteren Zeitpunkt erfolgen, da das Fläschchen gemäß einer bevorzugten Ausführungsform der Erfindung bereits durch das bloße Aufsetzen des Verschlusses flüssigkeitsdicht abgedichtet ist. Die Sterilbarriere bei steril abgefüllten Flüssigkeiten bildet die Metallfolie.

Die gasdichte Abdichtung nach dem induktiven Versiegeln ermöglicht eine anschließende Sterilisierung auch mit einem giftigen Gas wie Ethylenoxid, ohne dass die Gefahr besteht, dass das Gas in das Fläschchen eintritt.

Das Befüllen von Pharmafläschchen erfolgt in der Regel in einem aseptischen Anlagenbereich. Aus Kosten- sowie aus Sicherheitsgründen versucht man, diesen Anlagenbereich in der Regel möglichst klein zu halten.

Die Erfindung ermöglicht es, das induktive Versiegeln in einen nach dem aseptischen Bereich angeordneten Bereich durchzuführen.

Das induktive Versiegeln kann insbesondere auch derart schnell erfolgen, dass die Fläschchen auf dem Band nicht angehalten werden müssen, sondern dass diese während des Vorbeilaufens an der Anlage kontaktlos versiegelt werden.

Neben einer gasdichten Abdichtung und als etwaige Sterilbarrieren wirkt die Metallfolie, insbesondere die Aluminiumfolie, auch als Barriere für organische Lösungsmittel und verhindert insbesondere das Herauslösen von Lösungsmitteln, etwa aus der elastischen Dichtung.

### Beschreibung der Zeichnungen

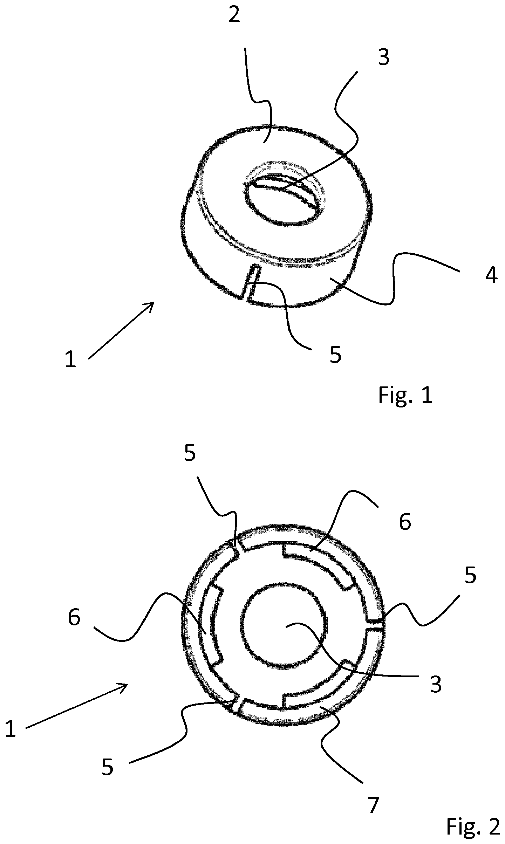

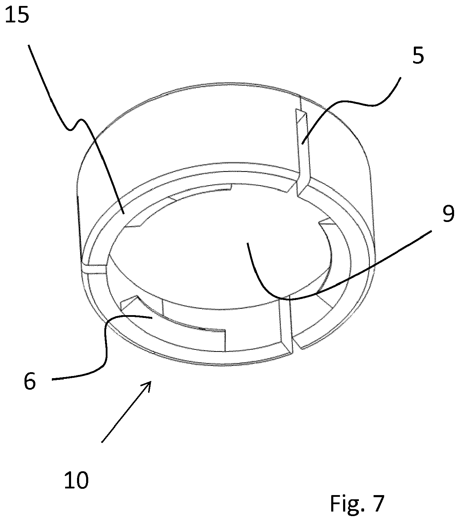

Die Erfindung soll im folgenden Bezug nehmend auf die Zeichnungen Fig. 1 bis Fig. 10 unter Bezugnahme auf beispielhafte Ausführungsformen näher erläutert werden.

Fig. 1 zeigt eine perspektivische Ansicht einer Kappe 1, welche für einen erfindungsgemäßen Verschluss vorgesehen ist.

Die Kappe 1 ist als Prellverschluss ausgebildet und hat einen im Wesentlichen kreisförmigen Querschnitt.

Die Oberseite 2 der Kappe ist mit einer mittigen Aussparung 3 versehen, durch welche beispielsweise eine Hohlnadel in den Verschluss eingeführt werden kann.

Die neben der Aussparung 3 vorhandene Oberseite 2 der Kappe sollte sich aber zumindest über den Rand des Fläschchens (nicht dargestellt) erstrecken.

Von der Grundform ist die Kappe 1 kreiszylindrisch ausgebildet.

Die Seitenwand 4 der Kappe 1 ist mit zumindest einem Schlitz 5 versehen, was dazu führt, dass die einzelnen Segmente der Seitenwand 4 sich beim Aufsetzen besser nach außen biegen können.

Aluminiumfolie und elastische Dichtung sind in dieser Ansicht nicht eingesetzt.

Fig. 2 zeigt die Unterseite der in Fig. 1 dargestellten Kappe 1. Zu erkennen ist die zentral angeordnete Aussparung 3. Weiter sind in dieser Ansicht drei Krallen 6 zu erkennen, welche von der Unterseite 7 der Seitenwand aus gesehen sich nach innen erstrecken und welche im montierten Zustand unter dem Kragen eines Fläschchens einrasten.

Die Krallen 6 nehmen insgesamt weniger als 180° des Umfangs der Kappe 1 ein.

Dies hat u.a. den Vorteil, dass die Kappe 1 beim Herstellen im Spritzguss durch Drehung der Kappe entformt werden kann.

Die Krallen 6 sind gleichmäßig über den Umfang verteilt und zwischen jeweils zwei Krallen befindet sich ein Schlitz 5, welcher in diesem Ausführungsbeispiel zumindest die Hälfte der Höhe der Seitenwand (4 in Fig. 1) einnimmt.

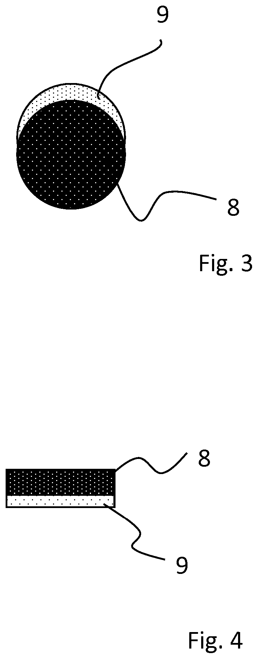

Fig. 3 zeigt schematisch eine elastische Dichtung 8, beispielsweise aus Silikon sowie eine Aluminiumfolie 9, welche in die Kappe eingesetzt werden.

Elastische Dichtung 8 und Aluminiumfolie 9 sind nicht fest miteinander verbunden, etwa verklebt.

Wie in Fig. 4 zu erkennen, werden elastische Dichtung 8 und Aluminiumfolie 9 übereinander gelegt und überdecken beide sowohl den Rand eines Fläschchens, auf das diese aufgesetzt werden, als auch die gesamte Öffnung des Fläschchens.

Die Aluminiumfolie 9 ist auf ihrer Unterseite mit einem Klebstoff versehen (nicht dargestellt).

So bildet die Aluminiumfolie ein induktiv aufbringbares Siegel derart, dass die Aluminiumfolie induktiv erwärmt wird, so dass der Kleber aufschmilzt und eine formschlüssige Verbindung vom Rand des Fläschchens und der Aluminiumfolie ermöglicht.

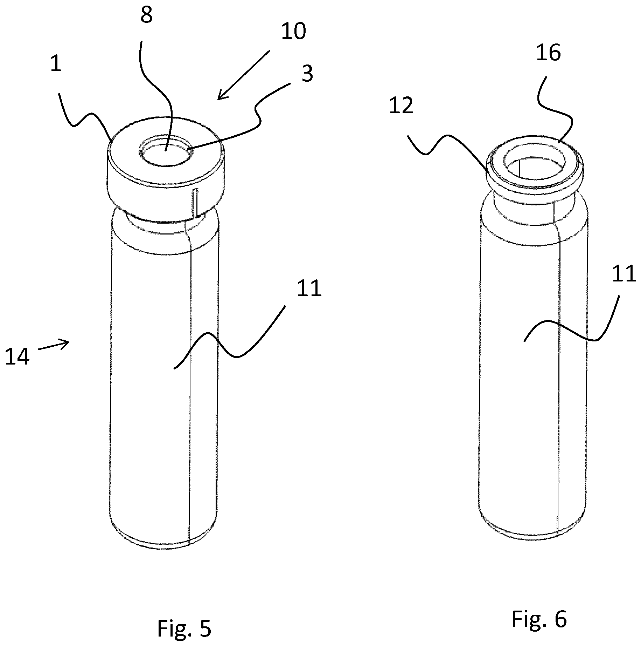

Fig. 5 zeigt eine perspektivische Ansicht eines Gebindes 14 für eine Flüssigkeit (nicht dargestellt).

Das Gebinde 14 umfasst ein in diesem Ausführungsbeispiel kreiszylinderförmiges Fläschchen 11 sowie den Verschluss 10, welcher die in den Figuren Fig. 1 und Fig. 3 dargestellte Kappe 1 sowie eine Aluminiumfolie und eine elastische Dichtung 8 umfasst.

Fig. 6 zeigt das Fläschchen 11 mit abgenommener Kappe.

Zu erkennen ist, dass das Fläschchen einen Kragen 12 aufweist.

Die Oberseite des Kragens 12 definiert den Rand, auf dem im montierten Zustand die Aluminiumfolie aufsitzt.

Auf der Unterseite der Kragens 12 rasten die Krallen der Kappe ein.

Fig. 7 zeigt eine perspektivische Ansicht eines Verschlusses.

Dieser umfasst die in Fig. 1 und Fig. 2 dargestellte Kappe, deren Seitenwand Schlitze 5 aufweist sowie angrenzend zur Unterseite 15 Krallen aufweist, um unter dem Kragen eines Fläschchens zu verrasten.

Hier sind nunmehr elastische Dichtung und Aluminiumfolie 9 eingesetzt. Zu erkennen ist die Aluminiumfolie.

Aluminiumfolie 9 und elastische Dichtung (nicht zu sehen) werden nach dem Einsetzen durch die Krallen 6 in der Kappe gehalten.

Der erfindungsgemäße Verschluss kann mithin als vormontiertes Bauteil in eine Anlage eingebracht werden und kann in einem Arbeitsschritt automatisiert aufgesetzt werden.

Die Krallen 6 sind, wie in dieser Ansicht gut zu erkennen, als nach innen ragende Keile ausgebildet, welche der kreisrunden Form der Kappe folgen.

Weiter sind die keilförmigen Krallen 6 auf ihrer Unterseite abgeschrägt, um beim Aufsetzen über den Kragen eines

Fläschchens zu gleiten. Dabei kann aufgrund der Schlitze 5 die Seitenwand sich leicht aufbiegen.

Auf der Oberseite verlaufen die Krallen in diesem Ausführungsbeispiel dagegen im wesentlichen horizontal.

Diese Ausgestaltung hat zur Folge, dass die Kappe beim gewaltsamen Abziehen zerstört wird.

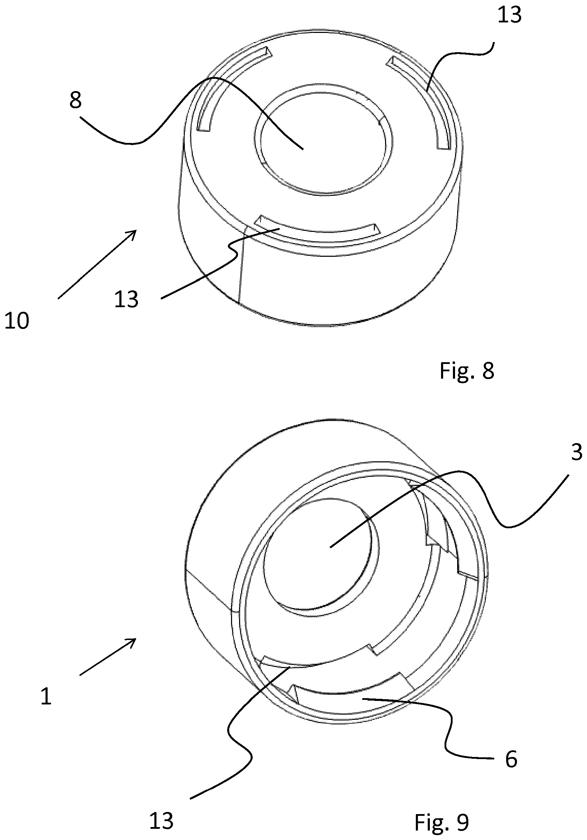

Fig. 8 zeigt eine alternative Ausführungsform eines Verschlusses.

Dieser entspricht im Wesentlichen dem zuvor dargestellten Verschluss mit dem Unterschied, dass auf der Oberseite Ausnehmungen 13 vorhanden sind, welche oberhalb der Krallen positioniert sind.

Die Ausnehmungen folgen dem kreisförmigen Verlauf der Kappe.

Weiter weist diese Ausführungsvariante keine Schlitze in der Seitenwand auf.

Bei dieser Ausführungsform muss ein geeignetes Material mit einer erhöhten Streckgrenze gewählt werden.

Durch die Aussparung der Kappe zu erkennen ist die elastische Dichtung 8.

Es versteht sich aber, dass es denkbar ist, oberhalb der elastischen Dichtung noch weitere Schichten anzubringen, ohne dass hierdurch die Funktion des Verschlusses beeinträchtigt wird.

Fig. 9 zeigt eine perspektivische Ansicht der Unterseite der Kappe 1, welche für den in Fig. 8 dargestellten Verschluss verwendet wurde.

Zu erkennen ist die mittige Aussparung. Weiter zu erkennen sind die Krallen 6, welche der zuvor dargestellten Ausführungsvariante entsprechen und welche nur einen Teil des Umfangs der Kappe einnehmen.

Neben der Funktion zur Entformung der Kappe haben Krallen, welche sich nicht über den gesamten Umfang erstrecken, den Vorteil, dass die Gefahr des Einschließens von Gas verringert wird. Beim Sterilisieren kann das Gas so ungehindert zwischen Kappe und Fläschchen treten und sodann wieder entweichen. Die Gasbarriere bildet allein das Aluminiumsiegel.

Zu erkennen ist hier des Weiteren, dass die Aussparungen 13, welche oberhalb der Krallen 6 angeordnet sind, von ihrer Form und Lage im Wesentlichen mit den Krallen 6 korrespondieren.

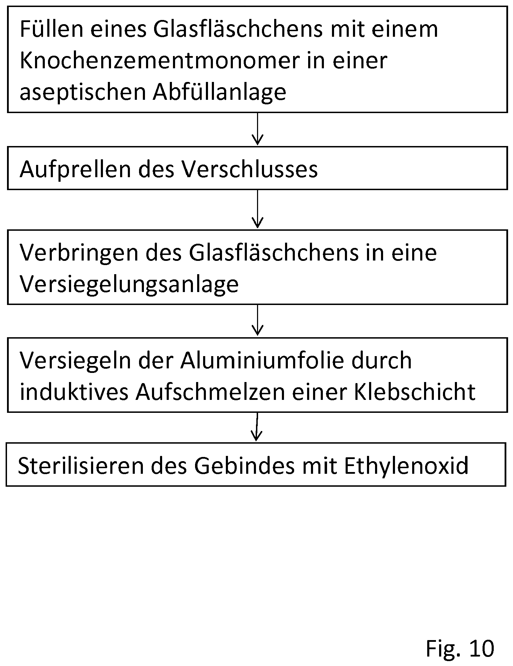

Bezug nehmend auf das Flussdiagramm gemäß Fig. 10 soll beispielhaft die Verwendung der erfindungsgemäßen Kappe zum Verschließen eines Fläschchens erläutert werden.

Zunächst wird ein Glasfläschchen mit einem Knochenzementmonomer in einer aseptischen Abfüllanlage befüllt und in der aseptischen Abfüllanlage der erfindungsgemäße Verschluss, beispielsweise der zuvor dargestellte Verschluss, aufgeprellt.

Das Aufprellen erfolgt automatisiert.

Sodann wird das Glasfläschchen beispielsweise auf einem Band in eine Versiegelungsanlage zum induktiven Versiegeln des Aluminiumsiegels verbracht. Diese Versiegelungsanlage braucht nicht zwingend im aseptischen Bereich angeordnet zu sein, da das Fläschchen nach dem Aufprellen des Verschlusses bereits flüssigkeitsdicht verschlossen ist und weitestgehend eine Sterilbarriere darstellt.

Sodann wird die Aluminiumfolie durch induktives Aufschmelzen der Klebschicht versiegelt und bildet nunmehr eine Gasbarriere, die sowohl das Eindringen von Gas verhindert als auch sicherstellt, dass leicht flüchtige Stoffe für einen langen Zeitraum aufbewahrt werden können.

Anschließend wird das Gebinde vor dem Verpacken mit Ethylenoxid sterilisiert.

Durch die Erfindung konnte erstmals ein Gebinde mit einem Septum für eine Einstichkanüle bereitgestellt werden, in welchem sich auch leicht flüchtige Stoffe für einen langen Zeitraum aufbewahren lassen.

### Bezugszeichenliste

- 1: Kappe
- 2: Oberseite
- 3: Aussparung
- 4: Seitenwand
- 5: Schlitz
- 6: Kralle
- 7: Unterseite
- 8: elastische Dichtung
- 9: Aluminiumfolie
- 10: Verschluss
- 11: Fläschchen
- 12: Kragen
- 13: Ausnehmung
- 14: Gebinde
- 15: Unterseite
- 16: Rand

## Patentansprüche

1. Verschluss (10), insbesondere für pharmazeutische Gebinde (14), umfassend eine auf ein Fläschchen (11) aufsetzbare Kappe (1) aus einem dielektrischen Material,
eine Metallfolie, welche im angebrachten Zustand auf einem Rand (16) des Fläschchens (11) sitzt,
wobei die Metallfolie zumindest auf der auf dem Rand (16) sitzenden Seite mit einem induktiv über die Metallfolie aufschmelzbaren Kleber versehen ist, und wobei über der Metallfolie eine elastische Dichtung (8) eingesetzt ist, und wobei die Kappe (1) eine Oberseite (2) mit zumindest einer Aussparung (3) aufweist, durch welche die elastische Dichtung (8)
und die Metallfolie durchstoßen werden kann, wobei der Verschluss (10) als Prellverschluss ausgebildet ist, **dadurch gekennzeichnet, dass**
die elastische Dichtung (8) eine Dicke zwischen 0,1 mm und 5 mm aufweist, so dass die Metallfolie nach dem Aufsetzen der Kappe (1) auf das Fläschchen (11) bereits dichtend auf den Rand (16) des Fläschchens (11) gedrückt ist, ohne dass der Kleber aufgeschmolzen wurde.

2. Verschluss nach dem vorstehenden Anspruch, **dadurch gekennzeichnet, dass** der Prellverschluss derart ausgebildet ist, dass er zumindest ohne Verwendung eines Werkzeugs nicht zerstörungsfrei abnehmbar ist.

3. Verschluss nach einem der vorstehenden Ansprüche, **dadurch gekennzeichnet, dass** die Metallfolie eine Aluminiumfolie (9) ist.

4. Verschluss nach einem der vorstehenden Ansprüche,
**dadurch gekennzeichnet, dass** die elastische Dichtung (8) gegenüber der Metallfolie verdrehbar ist.

5. Verschluss nach einem der vorstehenden Ansprüche,
**dadurch gekennzeichnet, dass** die Kappe (1) und/oder die elastische Dichtung (8) aus einem transparenten Material besteht.

6. Verschluss nach einem der vorstehenden Ansprüche,
**dadurch gekennzeichnet, dass** die Kappe (1) Krallen (6) zum Verrasten auf einem Kragen (12) des Fläschchens (11) aufweist, wobei die Krallen (6) insgesamt über einen Umfang der Kappe (1) von maximal 180° verteilt sind.

7. Verschluss nach einem der vorstehenden Ansprüche,
**dadurch gekennzeichnet, dass** die Kappe (1) Krallen (6) zum Verrasten auf einem Kragen (12) des Gebindes aufweist, wobei die Kappe (1) auf ihrer Oberseite (2) über den Krallen (6) Ausnehmungen (13) aufweist.

8. Verschluss nach einem der vorstehenden Ansprüche,
**dadurch gekennzeichnet, dass** die Kappe (1) Krallen (6) zum Verrasten auf einem Kragen (12) des Fläschchens (11) aufweist, wobei die Metallfolie und die elastische Dichtung (8) von den Krallen (6) in der Kappe (6) gehalten sind.

9. Gebinde (14), umfassend ein Fläschchen (11) mit einem Verschluss (10) nach einem der vorstehenden Ansprüche.

10. Gebinde nach dem vorstehenden Anspruch, **dadurch**
**gekennzeichnet, dass** das Gebinde (14) mit einem Monomer für eine Autopolymerisation gefüllt ist, insbesondere mit einem Knochenzementmonomer.

11. Mischvorrichtung für Knochenzement, umfassend ein mit
Knochenzementmonomer gefülltes Gebinde (14) nach dem vorstehenden Anspruch.

12. Verfahren zum Verschließen eines Fläschchens (11) mit
einem Verschluss (10) umfassend eine auf das Fläschchen (11) aufsetzbare Kappe (1) aus einem dielektrischen Material,
eine Metallfolie, welche im angebrachten Zustand auf einem Rand (16) des Fläschchens (11) sitzt,
wobei die Metallfolie zumindest auf der auf dem Rand (16) sitzenden Seite mit einem induktiv über die Metallfolie aufschmelzbaren Kleber versehen ist,
und wobei über der Metallfolie eine elastische Dichtung (8) mit einer Dicke zwischen 0,1 mm und 5 mm eingesetzt ist, und wobei die Kappe (1) eine Oberseite (2) mit zumindest einer Aussparung (3) aufweist, durch welche die elastische Dichtung (8) und die Metallfolie durchstoßen werden kann, wobei das Fläschchen (11) befüllt wird, sodann der Verschluss (10) aufgesetzt wird und im Anschluss mittels Induktion die Metallfolie erwärmt wird, so dass der Kleber aufschmitzt und die Metallfolie mit einem Rand (16) des Fläschchens (11) stoffschlüssig verbindet, **dadurch gekennzeichnet, dass** das induktive Erwärmen der Metallfolie nach Verlassen eines aseptischen Abfüllbereiches erfolgt.

13. Verfahren zum Verschließen eines Fläschchens nach dem vorstehenden Anspruch, **dadurch gekennzeichnet, dass** das Fläschchen äußerlich (11) nach dem stoffschlüssigen Verbinden von Metallfolie und Rand (16) mit einem Gas sterilisiert wird.

## Claims

1. A closure (10), in particular for pharmaceutical packages (14), comprising:
a cap (1) made of a dielectric material and attachable on a vial (11);
a metal foil which in its assembled state rests on a rim (16) of said vial (11);
wherein at least on the surface that rests on the rim (16) the metal foil is provided with an adhesive which is meltable inductively through the meta! foil; and
wherein an elastic seal (8) is inserted above the metal foil; and wherein the cap (1) has a base wall (2) with at least one opening (3) through which the elastic seal (8) and the metal foil can be perforated;
wherein the closure (10) is in the form of a snap-on closure;
**characterised in that**
the elastic seal (8) has a thickness between 0.1 mm and 5 mm so that, once the cap (1) has been applied on the vial (11), the metal foil is already sealingly pressed onto the rim (16) of the vial (11) without the adhesive having been melted yet.

2. The closure according to the preceding claim, wherein the snap-on closure is designed so that it cannot be removed without being destroyed, at least not without using a tool.

3. The closure according to any one of the preceding claims, **characterised in that** the metal foil is an aluminium foil (9).

4. The closure according to any one of the preceding claims, **characterised in that** the elastic seal (8) is rotatable relative to the metal foil.

5. The closure according to any one of the preceding claims, **characterised in that** the cap (1) and/or the elastic seal (8) is/are made of a transparent material.

6. The closure according to any one of the preceding claims, **characterised in that** the cap (1) has claws (6) for being latched on a collar (12) of the vial (11), wherein the claws (6) are distributed around the circumference of the cap (1) covering a maximum of 180° in total.

7. The closure according to any one of the preceding claims, **characterised in that** the cap (1) has claws (6) for being latched on a collar (12) of the vial, wherein the cap (1) has openings (13) in its base wall (2) above the claws (6).

8. The closure according to any one of the preceding claims, **characterised in that** the cap (1) has claws (6) for being latched on a collar (12) of the vial (11), wherein the metal foil and the elastic seal (8) are retained in the cap by the claws (6).

9. A package (14) comprising a vial (11) with a closure (10) according to any one of the preceding claims.

10. The package according to the preceding claim, **characterised in that** the package (14) is filled with a monomer for autopolymerisation, in particular with a bone cement monomer.

11. A mixing device for bone cement, comprising a package (14) according to the preceding claim, which is filled with bone cement monomer.

12. A method for sealing a vial (11) with a closure (10) that comprises a cap (1) made of a dielectric material and attachable on the vial (11);
a metal foil which in its assembled state rests on a rim (16) of the vial (11);
wherein at least on the surface that rests on the rim (16) the metal foil is provided with an adhesive which is meltable inductively through the metal foil; and
wherein an elastic seal (8) with a thickness between 0.1 mm and 5 mm is inserted above the metal foil; and wherein the cap (1) has a base wall (2) with at least one opening (3) through which the elastic seal (8) and the metal foil can be perforated;
wherein the vial (11) is filled, then the closure (10) is applied and subsequently the metal foil is heated by induction such that the adhesive melts thereby creating a material bond between the metal foil and a rim (16) of the vial (11);
**characterised in that**
the inductive heating of the meta! foil is performed after leaving an aseptic filling area.

13. The method for sealing a vial according to the preceding claim, **characterised in that** after the material bond has been created between the metal foil and the rim (16), the vial (11) is sterilized externally using a gas.

## Revendications

1. Fermeture (10), destinée en particulier à des contenants (14) pharmaceutiques, comprenant un capuchon (1) pouvant être rapporté sur un flacon (11) et constitué d'un matériau diélectrique,
une feuille métallique qui, à l'état monté, repose sur un bord (16) du flacon (11),
la feuille métallique étant dotée, au moins sur la face posée sur le bord (16), d'un adhésif pouvant être mis en fusion par induction, par l'intermédiaire de la feuille métallique,
et un joint d'étanchéité (8) élastique étant inséré au-dessus de la feuille métallique, et le capuchon (1) présentant une face supérieure (2) avec au moins un évidement (3) à travers lequel le joint (8) élastique et la feuille métallique peuvent être percés, la fermeture (10) étant réalisée sous forme de fermeture encliquetable,
**caractérisée en ce que**
le joint (8) élastique présente une épaisseur comprise entre 0,1 mm et 5 mm, de sorte qu'après la mise en place du capuchon (1) sur le flacon (11), la feuille métallique est déjà pressée de manière étanche sur le bord (16) du flacon (11), sans que l'adhésif ait été appliqué par fusion.

2. Fermeture selon la revendication précédente, **caractérisée en ce que** la fermeture encliquetable est conçue de manière à ce qu'elle ne puisse pas être retirée sans être détruite, du moins pas sans utiliser un outil.

3. Fermeture selon l'une des revendications précédentes, **caractérisée en ce que** la feuille métallique est une feuille d'aluminium (9).

4. Fermeture selon l'une des revendications précédentes, **caractérisée en ce que** le joint (8) élastique peut être tourné par rapport à la feuille métallique.

5. Fermeture selon l'une des revendications précédentes, **caractérisée en ce que** le capuchon (1) et/ou le joint (8) élastique est constitué d'un matériau transparent.

6. Fermeture selon l'une des revendications précédentes, **caractérisée en ce que** le capuchon (1) présente des griffes (6) en vue d'un encliquetage sur un collet (12) du flacon (11), les griffes (6) étant réparties globalement sur un pourtour du capuchon (1) d'au maximum 180°.

7. Fermeture selon l'une des revendications précédentes, **caractérisée en ce que** le capuchon (1) présente des griffes (6) en vue d'un encliquetage sur un collet (12) du contenant, le capuchon (1) comportant des évidements (13) sur sa face supérieure (2), au-dessus des griffes (6).

8. Fermeture selon l'une des revendications précédentes, **caractérisée en ce que** le capuchon (1) présente des griffes (6) en vue d'un encliquetage sur un collet (12) du flacon (11), la feuille métallique et le joint (8) élastique étant maintenus dans le capuchon (1) par les griffes (6).

9. Contenant (14), comprenant un flacon (11) doté d'une fermeture (10) selon l'une des revendications précédentes.

10. Contenant selon la revendication précédente, **caractérisé en ce que** le contenant (14) est rempli avec un monomère destiné à une auto-polymérisation, en particulier avec un monomère de ciment pour os.

11. Dispositif de mélange pour ciment pour os, comprenant un contenant (14) rempli avec un monomère de ciment pour os, selon la revendication précédente.

12. Procédé de fermeture d'un flacon (11) avec une fermeture (10) comprenant un capuchon (1) pouvant être rapporté sur le flacon (11) et constitué d'un matériau diélectrique,
une feuille métallique qui, à l'état monté, repose sur un bord (16) du flacon (11),
la feuille métallique étant dotée, au moins sur la face posée sur le bord (16), d'un adhésif pouvant être mis en fusion par induction, par l'intermédiaire de la feuille métallique,
et un joint d'étanchéité (8) élastique d'une épaisseur comprise entre 0,1 mm et 5 mm étant inséré au-dessus de la feuille métallique, et le capuchon (1) présentant une face supérieure (2) avec au moins un évidement (3) à travers lequel le joint (8) élastique et la feuille métallique peuvent être percés, sachant que le flacon (11) est rempli, puis la fermeture (10) est mise en place et ensuite la feuille métallique est chauffée par induction, de sorte que l'adhésif fond et relie la feuille métallique par adhérence à un bord (16) du flacon (11), **caractérisé en ce que** le chauffage par induction de la feuille métallique a lieu après avoir quitté une zone de remplissage aseptique.

13. Procédé de fermeture d'un flacon selon la revendication précédente, **caractérisé en ce que** le flacon (11) est stérilisé à l'extérieur avec un gaz après la liaison par adhérence entre la feuille métallique et le bord (16).
